# EUROPEAN PATENT APPLICATION

(11) **EP 4 775 236 A1**
(43) Date of publication of application: **15.07.2026**
(21) Application number: 25151531.8
(22) Date of filing: 13.01.2025
(51) Int. Cl.: A61M 5/142

(54) **MEDICAL DEVICES**

(71) Applicant: NXP B.V., 5656 AG Eindhoven (NL)
(72) Inventor: Snijder,, Bart, 5656 AG Eindhoven (NL); Piqué,, Gerard Villar, 5656 AG Eindhoven (NL); Neuteboom,, Harry, 5656 AG Eindhoven (NL)
(74) Representative: Schwarzweller, Thomas

(57) **Abstract**

A medical device comprising: a pump unit, wherein the pump unit is configured to control a pump such that the pump is configured to deliver a treatment to a user as required; a power supply unit comprising one or more batteries; an emergency unit configured to perform an emergency procedure if a fault is detected in one or more circuits of the medical device; and a capacitor; wherein the power supply unit is configured to charge the capacitor; the capacitor is configured to power the pump unit; and the capacitor is configured to provide power to the emergency unit.

## Description

### Field

The present disclosure relates to medical devices. In particular, the present disclosure relates to medical devices including a pump unit, for example insulin delivery systems.

### Summary

According to a first aspect of the present disclosure there is provided a medical device comprising: a pump unit, wherein the pump unit is configured to control a pump such that the pump is configured to deliver a treatment to a user as required; a power supply unit comprising one or more batteries; an emergency unit configured to perform an emergency procedure if a fault is detected in one or more circuits of the medical device; and a capacitor; wherein: the power supply unit is configured to charge the capacitor; the capacitor is configured to power the pump unit; and the capacitor is configured to provide power to the emergency unit.

In one or more embodiments the emergency procedure comprises providing a notification to the user.

In one or more embodiments the emergency unit comprises an output device configured to provide the notification.

In one or more embodiments the emergency procedure comprises selectively disconnecting a circuit in the medical device that has been detected as containing a fault, wherein the circuit is any circuit apart from the emergency unit.

In one or more embodiments the emergency procedure comprises selectively disabling the pump.

In one or more embodiments the capacitor is a supercapacitor.

In one or more embodiments the power supply unit comprises a voltage regulator.

In one or more embodiments the voltage regulator is configured to: receive a regulator input voltage from the one or more batteries and provide a regulator output that is different from the regulator input voltage; and charge the capacitor using the regulator output.

In one or more embodiments the voltage regulator comprises a power converter.

In one or more embodiments: the power supply unit may comprise a pre-charge unit configured to charge the capacitor until the voltage across the capacitor reaches a pre-charge threshold amount; the voltage regulator may comprise a step-up power converter, such that the regulator output voltage is higher than the regulator input voltage; the voltage regulator may be configured to be disabled when the voltage across the capacitor is less than the pre-charge threshold amount; and the pre-charge unit may be configured to be disabled when the voltage across the capacitor is greater than the pre-charge threshold amount.

In one or more embodiments, the emergency unit comprises a system check unit configured to detect faults in the medical device.

In one or more embodiments, the emergency unit comprises an emergency system unit configured to perform the emergency procedure after a fault is detected by the system check unit.

In one or more embodiments the emergency unit is configured to perform the emergency procedure if a voltage provided by the one or more batteries falls below a predetermined battery threshold.

In one or more embodiments the emergency procedure comprises: isolating the capacitor from all circuits apart from the emergency unit.

In one or more embodiments the pump is a shape memory alloy wire drive pump.

In one or more embodiments the pump is an insulin pump.

In one or more embodiments the emergency procedure comprises continuing to operate the pump.

In one or more embodiments the capacitor has a capacitance of at least 1mF, 10mF, 25mF, 50mF or 75mF.

While the disclosure is amenable to various modifications and alternative forms, specifics thereof have been shown by way of example in the drawings and will be described in detail. It should be understood, however, that other embodiments, beyond the particular embodiments described, are possible as well. All modifications, equivalents, and alternative embodiments falling within the spirit and scope of the appended claims are covered as well.

The above discussion is not intended to represent every example embodiment or every implementation within the scope of the current or future Claim sets. The figures and Detailed Description that follow also exemplify various example embodiments. Various example embodiments may be more completely understood in consideration of the following Detailed Description in connection with the accompanying Drawings.

### Brief Description of the Drawings

One or more embodiments will now be described by way of example only with reference to the accompanying drawings in which:
Figure 1 shows an example medical device;
Figure 2 shows a medical device according to an embodiment of the present disclosure;
Figure 3 shows example operational plots of the medical device of Figure 2, in the event that no fault is detected;
Figure 4 shows a medical device according to another embodiment of the present disclosure; and
Figure 5 shows example operational plots of the medical device of Figure 4, in the event that a fault is detected.

### Detailed Description

Throughout this disclosure (on-body) insulin delivery systems are used as an example, but it will be understood that the techniques disclosed herein may apply to other types of medicines and / or medical devices. Especially those which include a pump, or any other device which is battery operated with critical functionality.

An exemplary insulin delivery system is a battery-operated medical device that uses a shape-memory-alloy (SMA) driven pump to supply insulin to persons that suffer from diabetes in an automated or self-controlled way.

With regular insulin injection being of vital importance to the patient, proper functioning of such a device is of high importance. System robustness is critical and any possible malfunctioning of the system should be detected and recovered where possible. Furthermore, the end-user should always be alerted to any system malfunction which is detected.

Device manufacturers therefore often put a lot of effort into implementing functional safety which can include system self-checks, fallback mechanisms and / or redundancy, for example. In this way, the medical devices may be able to continue functional operation in case of detected system errors and to alert the patient by way of audible and / or visual warnings when proper system functionality cannot be guaranteed and system replacement is required.

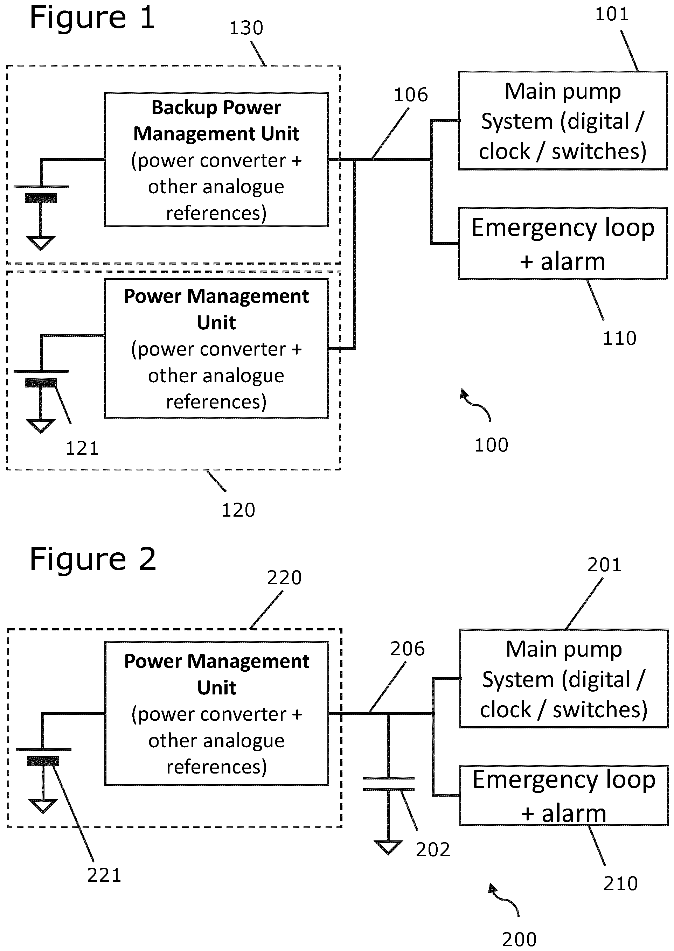

Figure 1 shows an example medical device 100, according to the above-discussed specifications. The medical device 100 includes a main pump system 101 which is configured to control a pump such that the pump is configured to deliver a treatment to a user as required. In the present example, the main pump system 101 contains the SMA wire drive for the insulin pump. The medical device 100 also includes an emergency unit 110 configured to perform an emergency procedure if a fault is detected in one or more circuits of the medical device 100. The emergency unit 110 may also contain one or more circuits for detecting possible errors within the medical device 100. It is critical for the emergency unit 110 to continue working at all times, especially in the case of errors in the functional circuitry. The medical device 100 also includes a power supply unit 120 which includes one or more batteries 121, configured to generate the main (regulated) supply.

Medical device pumps (such as insulin pumps) are typically operated such that a treatment is delivered periodically, for example at regular intervals or in response to a detection of a need for treatment such as excess glucose levels. These pumps also typically draw high currents during operation.

Because relatively high pump currents are often requested from a system running on one or more batteries, the power supply unit 120 of such a medical device 100 may need to be relatively complex. Adding redundancies for this power supply unit 120 through, for example extra batteries and/or circuitry 130 increases system robustness towards failures, but comes at the expense of increased system complexity, increased silicon area, possible increase of the number of required external components and an increase in weight (which may be significant in the case of adding backup batteries such as is shown in the figure).

When the emergency unit 110 and/or pump unit 101 are operating as independent blocks, functional safety, either through audible/visual or radio-transmitted alarms or possibly assisted through spare insulin pulses (injections), can be increased by having a sufficiently large backup supply at a regulated level (i.e., the voltage at a regulated node 106). Whenever errors are detected in the power supply unit, the emergency unit can be activated, running from this buffered regulated supply, hence being more robust to errors in the power infrastructure.

By dissipating electrical energy, SMA wire-drive pumps (such as the one used with the example medical device 100 shown in Figure 1) create physical movement through the heating of its SMA wires that consequently contract. For such SMA wires to have sufficient momentum, it is important to drive these with a minimum required voltage. While heating the SMA wires requires a significant amount of current, maintaining this as minimum driving voltage over the lifetime of a medical device 100 is a challenge when supplied with cheap and small batteries such as alkaline batteries, which indeed is often the case.

The present disclosure describes a means to increase the functional safety of medical devices by using stored energy in a capacitor as a main power source for the functional circuitry, and as a backup energy source to extend the duration of the emergency procedure. Through circuit isolation and direct supply of the emergency unit (the most critical block for the purpose of functional safety), the functional safety measures become more robust towards circuit errors in the pump unit and power supply unit or in the case of unexpected battery deep depletion. It is important to consider that safety (or alerting a user about the lack of safety) is of paramount importance for medical devices.

Since the electrical size (i.e., capacitance) of the output capacitor of a power converter is typically dimensioned to guarantee a certain output voltage ripple, in low-power battery operated devices very large capacitors are not considered, and so the added cost associated with a large capacitor is typically avoided. However, for the reasons discussed within this disclosure, the inventors have discovered that battery supplied SMA-wire drive pumps, and other medical devices considered by this disclosure, benefit from using large capacitors not only in the functional mode to maintain a stable driving supply but also for the extension of the functionality of critical emergency loops. By using the same capacitor for both purposes, the advantages of both may be gained without adding further expense. In particular, in the case of system malfunctions, using capacitors as disclosed herein can add significant value from a functional safety point of view, without further increasing the cost.

With treatment delivery systems often having a discrete behaviour over time (for example pump pulses or charge packages), using a power infrastructure incorporating a large storage capacitor prevents excessive voltage drops from occurring during treatment delivery. A large capacitor (for example, a capacitor with a capacitance of at least 1mF, 10mF, 25mF, 50mF or 75mF; and potentially with a capacitance of less than 5F) can be slowly charged between pump actions which minimises the peak currents drawn from the batteries. This enables a higher amount of energy to be extracted from the batteries over the course of their lifetimes. Such a large capacitor can be larger than one that would be suitable for merely reducing the size of a ripple in an output voltage. By using a large capacitor, the accumulated charge is sufficient to drive the SMA wires (or similar) with a low impedance, thereby guaranteeing a minimum driving voltage and proper pump action within the medical device.

Figure 2 shows a medical device 200 according to an embodiment of the present disclosure. The medical device 200 includes a pump unit 201, wherein the pump unit 201 is configured to control a pump (not shown) such that the pump is configured to deliver a treatment to a user as required. The medical device 200 also includes a power supply unit 220, which includes one or more batteries 221. The medical device 200 also includes an emergency unit 210 configured to perform an emergency procedure if a fault is detected in one or more circuits of the medical device 200, and a capacitor 202. In some embodiments, the capacitor 202 is a large capacitor. The capacitor 202 is connected between the regulated node 206 and a ground terminal.

The power supply unit 220 is configured to charge the capacitor 202, and the capacitor 202 is configured to power the pump unit 201. The capacitor 202 is also configured to provide power to the emergency unit 210. Advantageously, this allows the medical device 200 to provide sufficient power to operate the pump whilst minimizing peak currents from the one or more batteries 221 of the power supply circuit 220, thereby extending the useful lifetime of the batteries 221. Furthermore, the capacitor 202 being used to provide power to the emergency unit 210 allows the emergency unit 210 to continue to operate, thereby allowing the emergency unit 210 to perform an emergency procedure, in the event of a fault within the one or more batteries 221 or the power management unit 220 or a critically low battery condition (i.e., if the voltage provided by the one or more batteries falls below a predetermined battery threshold).

Because the amount of time which the capacitor 202 is able to power the emergency unit 210 without receiving charge from the power supply unit 220 (for example in the event of a fault within the power supply unit 220) is directly dependent on the amount of charge stored on the capacitor 202 at the occurrence of the fault, the advantages discussed above may be more significant if the capacitor 202 is a large capacitor. In some embodiments, the capacitor 202 is a supercapacitor, such that the capacitor 202 has good energy density and a large specific capacitance. For instance, a device which uses an electrostatic double-layer capacitance and electrochemical pseudocapacitance in order to store charge. In some embodiments, the capacitor 202 has a low equivalent series resistance (ESR). In these embodiments, the capacitor 202 may be well-suited to driving an SMA driven pump.

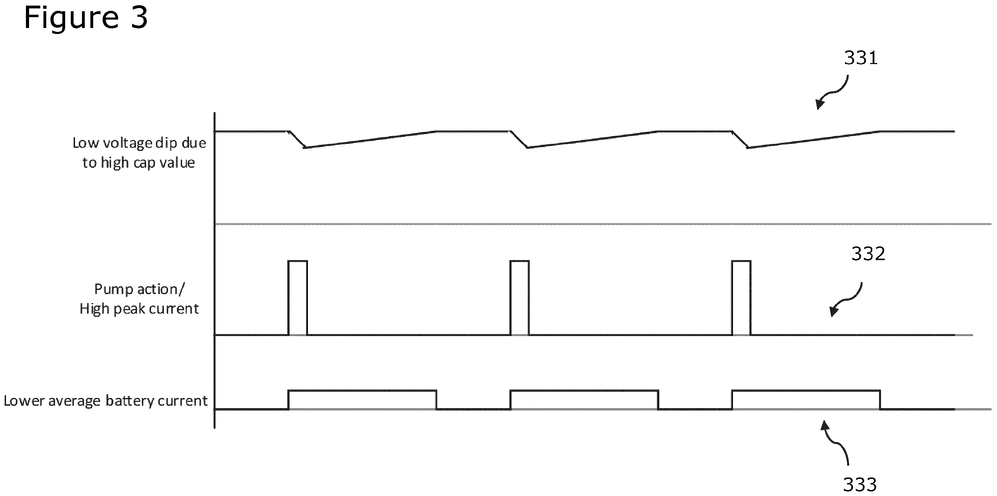

Figure 3 shows example operational plots of the medical device of Figure 2, in the event that no fault is detected. A first operational plot 331 shows capacitor voltage against time. Because the capacitor has a large capacitance, the voltage dips associated with pump operations are relatively low compared to the voltage across the capacitor. A second operational plot 332 shows pump current against time. As is apparent from the figure, the pump in this embodiment is operated using large and brief current pulses which are applied at regular intervals. Voltage drops corresponding to these pulses are also visible in the first operational plot 331. A third operational plot 333 shows battery current against time. As apparent from the first and third operational plots 331, 333, a relatively small current is drawn from the batteries in order to recharge the capacitor after a pump operation and therefore return the voltage across the capacitor back up to the level that is provided by the power management unit.

As mentioned above, the emergency unit is configured to perform an emergency procedure if a fault is detected in one or more circuits of the medical device. The emergency procedure may include one or more of the following:
- providing a notification to the user;
- selectively disconnecting a circuit in the medical device that has been detected as containing a fault. Such a circuit can be any circuit apart from the emergency unit;
- selectively disabling / disconnecting the pump from the capacitor;
- controlling the pump; and
- selectively disconnecting all circuits in the medical device, apart from the emergency unit and, in some examples, the pump, from the capacitor.

In examples wherein the emergency procedure includes providing a notification to the user, the notification may be auditory, visual, haptic, wireless (such as a text message, email or app notification) or any other suitable format. The emergency unit may include an output device configured to provide the notification to the user.

Driving the pump unit and emergency circuit from the capacitor increases the number of possibly erroneous circuits which may be isolated such that the remaining charge in the capacitor can be used to perform the emergency procedure. The emergency procedure may be continued until the fault is rectified, or until the capacitor is discharged below a critical level. In any case, the emergency capabilities of a medical device which uses a capacitor as such are greatly improved with respect to conventional devices.

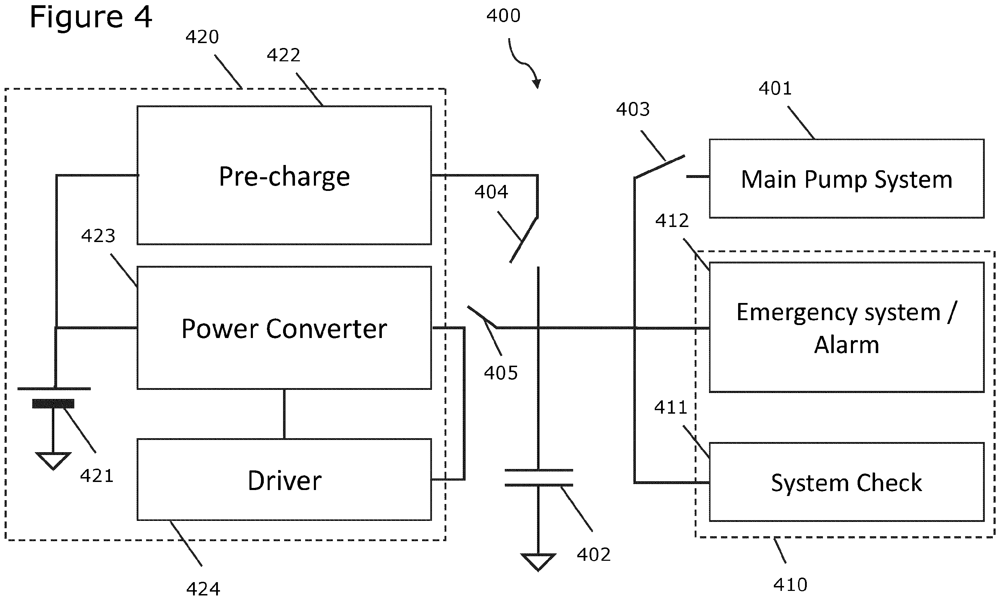

Figure 4 shows a medical device 400 according to an embodiment of the present disclosure. The medical device 400 includes a pump unit 401, wherein the pump unit 401 is configured to control a pump (not shown) such that the pump is configured to deliver a treatment to a user as required. The medical device 400 also includes a power supply unit 420, which includes one or more batteries 421. The medical device 400 also includes an emergency unit 410 configured to perform an emergency procedure if a fault is detected in one or more circuits of the medical device 400, and a capacitor 402.

In this embodiment, the capacitor 402 is connected to a regulated supply node, which is close to the critical circuitry (i.e., the pump unit 401 and emergency unit 410) with a potential to be isolated from malfunctioning circuitry. In this way, the capacitor 402 is able to act as a functional safety power supply for the emergency unit 410 whilst remaining largely independent to possible malfunctions in the medical device 400.

In this embodiment, the emergency unit 410 includes a system check unit 411 configured to detect faults in the medical device 400, and an emergency system unit 412 configured to perform the emergency procedure after a fault is detected in the medical device 400 by the system check unit 411. In other embodiments, faults may be detected within the medical device 400 by any other suitable means, for example each circuit within the medical device 400 may include an error detection mechanism.

In this embodiment, the power supply unit 420 includes a voltage regulator 423. In this embodiment, the voltage regulator 423 is a power converter, but in other embodiments, the voltage regulator 423 may be any other type of voltage regulator, for example a low dropout regulator. The voltage regulator 423 is configured to receive a regulator input voltage from the one or more batteries 421 and provide a regulator output with a voltage that is higher than the regulator input voltage, and charge the capacitor 402 using the regulator output. In this embodiment, because the voltage regulator 423 is a step-up power converter, the power supply unit 420 also includes a pre-charge unit 422 configured to charge the capacitor 402 until the voltage across the capacitor 402 reaches a pre-charge threshold amount. For example, the pre-charge threshold amount may be the maximum voltage output of the one or more batteries 421. In one or more embodiments, the voltage regulator 423 is configured to be disabled when the voltage across the capacitor 402 is less than the pre-charge threshold amount, and the pre-charge unit 422 is configured to be disabled when the voltage across the capacitor 402 is greater than the pre-charge threshold amount. In this way, the voltage regulator 423 may effectively replace the pre-charge unit 422 in the role of charging the capacitor 402 after the voltage across the capacitor has reached the pre-charge threshold amount. The voltage regulator 423 may be configured to charge the capacitor 402 until the voltage across the capacitor 402 reaches a target output voltage of the voltage regulator 423.

In this embodiment, the voltage regulator 423 is an up converter, but in other embodiments, a (switched or continuous-time) down or up / down converter may be used. In any case, the regulator output voltage may be lower or higher than the regulator input voltage. In some embodiments, the regulator output voltage may be the same as the regulator input voltage. The pre-charge unit 422 in Figure 4 is shown as a separate circuit to the voltage regulator 423, although in other examples the pre-charge unit 422 may be a part of the voltage regulator 423 architecture.

In this embodiment, the medical device 400 includes a first switch 403 configured to selectively disconnect the pump unit 401 from the capacitor 402, a second switch 404 configured to selectively disconnect the pre-charge unit 422 from the capacitor 402, and a third switch 405 configured to selectively disconnect the voltage regulator 423 from the capacitor 402, in the event that a fault is detected in each respective circuit. In some embodiments, the first switch 403 may be part of the pump, such that the first switch 403 may be configured to connect / disconnect the SMA wires of the pump (or equivalent). In some other embodiments, the medical device 400 includes one switch configured to selectively disconnect the power supply unit 420 from the capacitor 402 in the event that a fault is detected in the power supply unit 420. In yet further embodiments, the medical device 400 contains any other suitable arrangement of switches 403, 404, 405 configured to selectively disconnect a respective circuit within the medical device 400 from the capacitor 402 in the event that a fault is detected within that circuit. This means that, for example, the capacitor 402 can be isolated from the one or more batteries 421 (via a voltage regulator 423 and a pre-charge unit 422 in this example), which prevents the situation in which a faulty battery could drain the energy stored across the capacitor.

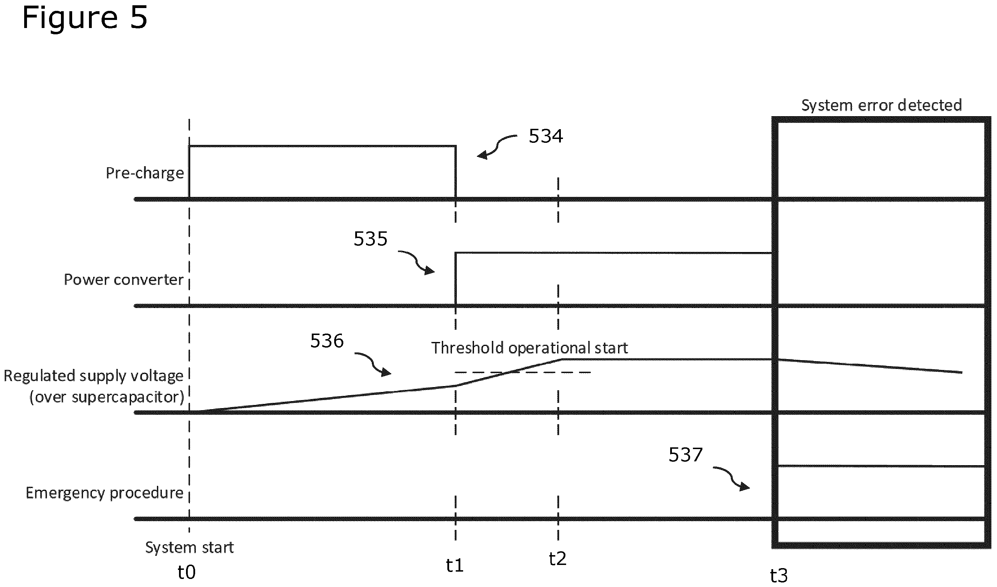

Figure 5 shows example operational plots of the medical device of Figure 4, from startup to shortly after a fault is detected in the voltage regulator. A first operational plot 534 shows a binary signal wherein a high signal indicates that the pre-charge unit is active and charging the capacitor, and a low signal indicates that the pre-charge unit is inactive and not charging the capacitor. A second operational plot 535 shows a binary signal wherein a high signal indicates that the voltage regulator is active and charging the capacitor, and a low signal indicates that the voltage regulator is not charging the capacitor. When the pre-charge unit and voltage regulator are inactive, they may be disabled. When the pre-charge unit or voltage regulator should be active they may be enabled. A third operational plot 536 shows the voltage across the capacitor against time. A fourth operational plot 537 shows a binary signal wherein a high signal indicates that an emergency procedure is active, and a low signal indicates that an emergency procedure is not active.

As described above, the pre-charge unit is configured to charge the capacitor until the voltage across the capacitor reaches the pre-charge threshold amount. This is represented in the figure by the period between t0 and t1. As also described above, the voltage regulator is configured to, after the voltage across the capacitor has reached the pre-charge threshold amount, charge the capacitor until the voltage across the capacitor reaches the target output voltage of the voltage regulator. This is represented in the figure by the period between t1 and t2.

In this embodiment, the pump unit is configured to control the pump after the voltage across the capacitor reaches a pump-control threshold amount, wherein the pump-control threshold amount is between the pre-charge threshold amount and the target output voltage of the voltage regulator. The pump-control threshold amount is represented by "Threshold operational start" in the figure and occurs at a time in between t1 and t2. In other embodiments, the power supply unit is configured to charge the capacitor until the voltage across the capacitor reaches the target output voltage of the voltage regulator, and the pump unit is configured to control the pump after the voltage across the capacitor has reached the target output voltage of the voltage regulator for the first time.

The period t2 to t3 shows normal functional operation of the medical device, in the absence of any detected errors. At time t3, during functional operation, an error is detected in the voltage regulator. When the error is detected, the emergency unit begins the emergency procedure. In this example, the emergency procedure includes initially selectively disconnecting all circuits in the medical device, apart from the emergency unit and the pump, from the capacitor. Therefore, after time t3 in this example, the fourth and fifth operational plots 534, 535 are low because the power supply unit and the pre-charge unit have been disconnected from the capacitor and as such, neither the pre-charge unit nor the voltage regulator are charging the capacitor. In addition, after time t3, the fourth operational plot 537 is high to indicate that the emergency unit is performing the emergency procedure and the third operational plot 536 shows the voltage across the capacitor gradually falling as the capacitor provides power to the emergency unit without receiving any additional charge.

In some other examples, if no faults are detected in the power supply unit or the one or more batteries, the emergency procedure may comprise disconnecting all circuits in the medical device apart from the emergency unit and the power supply unit, to ensure that the emergency unit remains functional for the maximum amount of time. In other examples, any other suitable emergency procedure may be used.

The instructions and/or flowchart steps in the above figures can be executed in any order, unless a specific order is explicitly stated. Also, those skilled in the art will recognize that while one example set of instructions/method has been discussed, the material in this specification can be combined in a variety of ways to yield other examples as well, and are to be understood within a context provided by this detailed description.

In some example embodiments the set of instructions/method steps described above are implemented as functional and software instructions embodied as a set of executable instructions which are effected on a computer or machine which is programmed with and controlled by said executable instructions. Such instructions are loaded for execution on a processor (such as one or more CPUs). The term processor includes microprocessors, microcontrollers, processor modules or subsystems (including one or more microprocessors or microcontrollers), or other control or computing devices. A processor can refer to a single component or to plural components.

In other examples, the set of instructions/methods illustrated herein and data and instructions associated therewith are stored in respective storage devices, which are implemented as one or more non-transient machine or computer-readable or computer-usable storage media or mediums. Such computer-readable or computer usable storage medium or media is (are) considered to be part of an article (or article of manufacture). An article or article of manufacture can refer to any manufactured single component or multiple components. The non-transient machine or computer usable media or mediums as defined herein excludes signals, but such media or mediums may be capable of receiving and processing information from signals and/or other transient mediums.

Example embodiments of the material discussed in this specification can be implemented in whole or in part through network, computer, or data based devices and/or services. These may include cloud, internet, intranet, mobile, desktop, processor, look-up table, microcontroller, consumer equipment, infrastructure, or other enabling devices and services. As may be used herein and in the claims, the following non-exclusive definitions are provided.

In one example, one or more instructions or steps discussed herein are automated. The terms automated or automatically (and like variations thereof) mean controlled operation of an apparatus, system, and/or process using computers and/or mechanical/electrical devices without the necessity of human intervention, observation, effort and/or decision.

It will be appreciated that any components said to be coupled may be coupled or connected either directly or indirectly. In the case of indirect coupling, additional components may be located between the two components that are said to be coupled.

In this specification, example embodiments have been presented in terms of a selected set of details. However, a person of ordinary skill in the art would understand that many other example embodiments may be practiced which include a different selected set of these details. It is intended that the following claims cover all possible example embodiments.

## Claims

1. A medical device comprising:
a pump unit, wherein the pump unit is configured to control a pump such that the pump is configured to deliver a treatment to a user as required;
a power supply unit comprising one or more batteries;
an emergency unit configured to perform an emergency procedure if a fault is detected in one or more circuits of the medical device; and
a capacitor; wherein:
the power supply unit is configured to charge the capacitor;
the capacitor is configured to power the pump unit; and
the capacitor is configured to provide power to the emergency unit.

2. The medical device of claim 1, wherein the emergency procedure comprises providing a notification to the user.

3. The medical device of claim 2, wherein the emergency unit comprises an output device configured to provide the notification.

4. The medical device of any preceding claim, wherein the emergency procedure comprises selectively disconnecting a circuit in the medical device that has been detected as containing a fault, wherein the circuit is any circuit apart from the emergency unit.

5. The medical device of any preceding claim, wherein the emergency procedure comprises selectively disabling the pump.

6. The medical device of any preceding claim, wherein the capacitor is a supercapacitor.

7. The medical device of any preceding claim, wherein the power supply unit comprises a voltage regulator configured to: receive a regulator input voltage from the one or more batteries and provide a regulator output that is different from the regulator input voltage; and charge the capacitor using the regulator output.

8. The medical device of claim 7, wherein the voltage regulator comprises a power converter.

9. The medical device of claim 7, wherein:
the power supply unit comprises a pre-charge unit configured to charge the capacitor until the voltage across the capacitor reaches a pre-charge threshold amount;
the voltage regulator comprises a step-up power converter, such that the regulator output voltage is higher than the regulator input voltage;
the voltage regulator is configured to be disabled when the voltage across the capacitor is less than the pre-charge threshold amount; and
the pre-charge unit is configured to be disabled when the voltage across the capacitor is greater than the pre-charge threshold amount.

10. The medical device of any preceding claim, wherein the emergency unit comprises:
a system check unit configured to detect faults in the medical device; and
an emergency system unit configured to perform the emergency procedure after a fault is detected by the system check unit.

11. The medical device of any preceding claim, wherein the emergency unit is configured to perform the emergency procedure if a voltage provided by the one or more batteries falls below a predetermined battery threshold.

12. The medical device of any preceding claim, wherein the emergency procedure comprises:
isolating the capacitor from all circuits apart from the emergency unit.

13. The medical device of any preceding claim, wherein the pump is a shape memory alloy wire drive pump, and optionally wherein the pump is an insulin pump.

14. The medical device of any preceding claim, wherein the emergency procedure comprises continuing to operate the pump.

15. The medical device of any preceding claim, wherein the capacitor has a capacitance of at least 1mF, 10mF, 25mF, 50mF or 75mF.
